# EUROPÄISCHE PATENTSCHRIFT

(11) **EP 1 241 943 B1**
(45) Veröffentlichungstag und Bekanntmachung des Hinweises auf die Patenterteilung: **02.03.2005**
(21) Anmeldenummer: 00977455.5
(22) Anmeldetag: 31.10.2000
(51) Int. Cl.: A01N 43/40, A01N 47/40, A01N 51/00, A01N 53/00

(54) **VERWENDUNG VON POLYSILOXANEN MIT QUARTÄREN AMINOGRUPPEN ALS FORMULIERUNGSHILFSMITTEL UND MITTEL ENTHALTEND DIESELBEN**
USE OF POLYSILOXANES CONTAINING QUATERNARY AMINO GROUPS AS FORMULATION AUXILIARY AGENTS, AND AGENTS CONTAINING THE SAME
UTILISATION DE POLYSILOXANES CONTENANT DES GROUPES AMINO QUATERNAIRES COMME AUXILIAIRES DE FORMULATION ET AGENTS LES CONTENANT

(30) Priorität: 12.11.1999 DE 19954394
(43) Veröffentlichungstag der Anmeldung: 25.09.2002
(73) Patentinhaber: Bayer HealthCare AG, 51368 Leverkusen (DE)
(72) Erfinder: SIRINYAN, Kirkor, 51467 Bergisch Gladbach (DE); HEINEN, Kerstin, 51429 Bergisch Gladbach (DE)
(86) Internationale Anmeldenummer: PCT/EP2000/010767
(87) Internationale Veröffentlichungsnummer: WO 2001/035739

(56) Entgegenhaltungen:
- EP-A- 0 436 359
- EP-A- 0 484 857
- EP-A- 0 710 688
- DE-A- 4 443 062
- DE-A- 19 807 630
- US-A- 4 342 742
- US-A- 5 846 997
- ZIOLOWSKY B: "Neue Haarbehandlungsmittel" SÖFW JOURNAL, Bd. 123, Dezember 1997 (1997-12), Seiten 822-824, XP002159447

## Beschreibung

Aus EP-A 0 017 121, 0 017 122, 0 282 720, 0 294 642, 0 166 122 und 0 164 668 ist eine Vielzahl von Polysiloxanen mit endständigen quartären Aminogruppen und deren Verwendung als sogenannte "Conditioner" in Haarwasch- und Haarpflegemitteln bekannt.

Bei der Herstellung von Wirkstoff-Formulierungen auf dem Gebiet der Pharmazie und der Veterinärmedizin stellt sich nun häufig und insbesondere bei wässrigen Formulierungen das Problem mangelnder Löslichkeit der Wirkstoffe und damit zusammenhängend auch einer mangelnden Lagerstabilität der fertigen Formulierungen.

Zur Anwendung zum Teil schwer wasserlöslicher Wirkstoffe in Form von dermal applizierbaren Flüssigformulierungen ist es notwendig, homogene Lösungen oder Emulsionen auf Basis von organischen Lösungsmitteln und insektiziden Wirkstoffen herzustellen. Dazu werden die Wirkstoffe zumeist in organischen Lösungsmitteln wie Isopropanol, 2-Butoxy-ethylacetat, Ethylenglykoldiacetat gelöst und gegebenenfalls mit weiteren Zusatzstoffen vermischt. Die Herstellung solcher Formulierungen ist in US 4 874 753, EP-A 137 627 und GB 2 135 886 beschrieben. Die Nachteile der besagten Systeme liegen z.B. bei Verwendung von Wirkstoffen aus der Klasse der Pyrethrine sowie Pyrethroide, insbesondere α-Cyanopyrethroide darin, dass sie zu schweren Hautirritationen führen und ferner eine geringe Langzeitwirkung aufweisen. Es ist wünschenswert, diese Formulierungen durch solche zu ersetzen, die hautverträglich sowie toxikologisch unbedenklich sind und sich durch eine Langzeitwirkung von mehreren Wochen auszeichnen.

Um den besagten Nachteil beispielsweise der bekannten Pyrethroide und Pyrethrine zu beheben wird in den Patentschriften AU-627 847, EP-A 413 610 vorgeschlagen, diese Wirkstoffe in hochsiedenden Lösungsmitteln wie Monopropylenglykol, die zusätzlich noch natürliche, hautverträgliche Öle wie Pinienöl, Sonnenblumenöl oder Sojaöl enthalten, zu lösen. Ferner kann der Patentschrift WO 91/13545 entnommen werden, dass gut wirksame, hautverträgliche Flüssigformulierungen hergestellt werden können, indem man die besagten Wirkstoffe in Mengen von >50% in aliphatischen Lösungsmitteln wie 2-(2-Butoxyethoxy)ethanol oder 2-(2-Methoxyethoxy)ethanol löst. Der Nachteil dieser Formulierungen liegt darin, dass sie den Einsatz größerer Wirkstoff-Mengen benötigen und zudem bei sensiblen Tierrassen zu Hautirritationen führen. Um durch Einsatz geringer Wirkstoffmengen eine akzeptable biologische Wirkung zu erreichen, wird in der Patentschrift US 5 466 458 die Verwendung von Emulsionen auf Basis der besagten Wirkstoffe mit den langkettigen, aliphatischen Aminen oder Alkoholen wie Hexadecan-1-ol, 1-Octadecylamine vorgeschlagen. Die Verwendung der langkettigen Amine hat den Nachteil, dass sie die besagten Wirkstoffe im Laufe der Zeit abbauen. Die Formulierungen auf Basis langkettiger Alkohole weisen in den meisten Fällen keine ausreichende Langzeitwirkung auf.

DE 198 07 630 offenbart die Verwendung von Silikonölen mit kationischer funktioneller Gruppen in wasserhaltiger Formulierungen enthaltend Nicotinyl Insektizide. Überraschenderweise werden diese Aufgaben durch die erfindungsgemäße Verwendung der oben genannten Polysiloxane gelöst. Es entstehen klare Lösungen oder Emulsionen von hoher Lagerstabilität.

Weiterhin erreicht man durch die erfindungsgemäße Verwendung dieser Polysiloxane erstaunlicherweise eine verbesserte Verträglichkeit und einen wirkungssteigernden, synergistischen Effekt, insbesondere in Verbindung mit Pyrethroiden und Pyrethrinen.

Die vorliegende Erfindung betrifft demnach die Verwendung von Polydimethylsiloxanen enthaltend mindestens eine quartäre Ammoniumgruppe als Formulierhilfsmittel in Formulierungen akarizider α-Cyanopyrethroide.

Weiterhin Gegenstand der vorliegenden Anmeldung sind neue Mittel, enthaltend
a) ein akarizides α-Cyanopyrethroid.
b) ein Polydimethylsiloxan enthaltend mindestens eine quartäre Ammoniumgruppe pro Molekül,
gegebenenfalls weitere Hilfs- und Trägerstoffe.

Die erfindungsgemäßen Mittel eignen sich insbesondere hervorragend zur Herstellung von Aerosol-, Pumpsprays, Spot on und Pour on's für den Einsatz bei der Parasitenbekämpfung am Tier.

Als geeignete Wirkstoffe hervorgehoben seien die Pyrethrine und Pyrethroide mit Common Names wie Fenvalerate [α-(p-Cl-phenyl)-isovaleriansäure-α-cyano-3-phenoxy-benzylester], Flumethrin [3-[2-(4-Chlorphenyl)-2-chlorvinyl]-2,2-dimethylcyclo-propancarbonsäure (α-cyano-4-fluor-3-phenoxy)-benzylester] und seine Enantiomere sowie Stereoisomere, Cyfluthrin [2,2-Dimethyl-3-(2,2-dichlorvinyl)-(α-cyano-4-fluor-3-phenoxy)-benzyl-ester], Permethrin [3-Phenoxybenzylcis, trans 3-(2,2-dichlorvinyl)-2,2-dimethylcyclopropancarboxylat], Cypermethrin [2,2-Dimethyl-3-(2,2-dichlorvinyl)-cyclopropancarbonsäure-α-cyano-3-phenoxy-benzylester], Deltamethrin [α-Cyano-3-phenoxybenzyl-cis, trans-3-(2,2-dibromvinyl)-2,2-dimethylcyclopropancarboxylat], Fluvalinate [2-Cyano-3- phenoxybenzyl-2(2-chlor-α,α,α-trifluor-p-toluido)-3-methylbutyrat eingesetzt werden, wobei Pyrethroide mit akarazider Wirkung zur Herstellung der neuen Formulierungen bevorzugt und die auf Basis von α-Cyanopyrethroiden wie Ester der α-Cyano-3-phenylbenzyl alkohole oder 4-Fluoro-α-Cyano-3-phenoxybenzylalkohole besonders zu bevorzugen sind. Der ganz besonders bevorzugte Wirkstoff ist Flumethrin.

Die Wirkstoff-Mengen können zwischen 0,1-15 % breit variiert werden. Die Mengen im Bereich 0,1-7,5 % sind bevorzugt. Die Mengen im Bereich 0,2 - 2,0 % werden zur Herstellung der erfindungsgemäßen, neuen Formulierungen besonders bevorzugt eingesetzt. Prozentangaben beziehen sich dabei auf Gewichtsprozent.

Selbstverständlich können in den erfindungsgemäßen Mitteln weitere Wirkstoffe als Kombinationspartner eingesetzt werden.

Als Kombinationswirkstoffe seien bevorzugt genannt die im Bereich zur Bekämpfung von ektoparasitierenden Insekten eingesetzten Insektizide wie Nicotinyl- sowie insbesondere Chlornicotinyl-Insektizide, N-Phenylpyrazole, Carbamate, Phosphor- und Phosphonsäureester, Wachstumshemmer bzw. Mischungen dieser Wirkstoffe untereinander sowie deren Mischungen mit Synergisten. Als Synergisten im Sinne dieser Anmeldung werden Verbindungen verstanden, die selbst nicht die gewünschte Wirksamkeit aufweisen, als Mischpartner jedoch zu einer Steigerung der Wirksamkeit der a.i. führen.

Als Chlomicotinyl-Insektizide seien genannt Verbindungen der Formeln (I),(II) und (III): 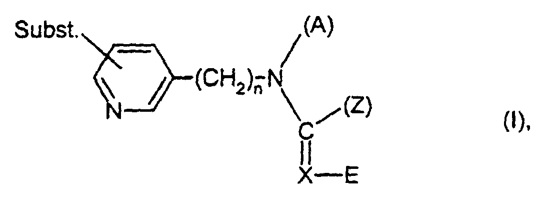 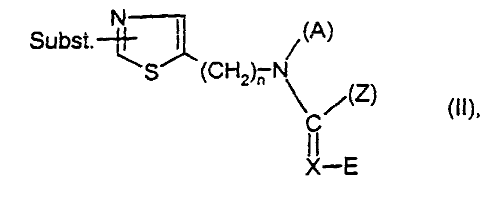 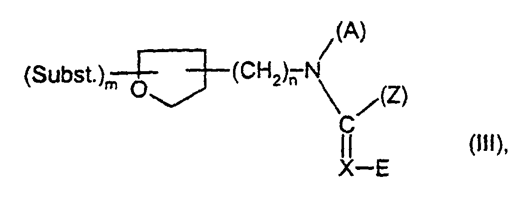 in welchen
- n: für 1 oder 2 steht,
- m: für 0, 1 oder 2 steht,
Subst. für einen der oben aufgeführten Substituenten, insbesonders für Halogen, ganz besonders für Chlor, steht,

A, Z, X und E die oben angegebenen Bedeutungen haben.

Im einzelnen seien folgende Verbindungen genannt:

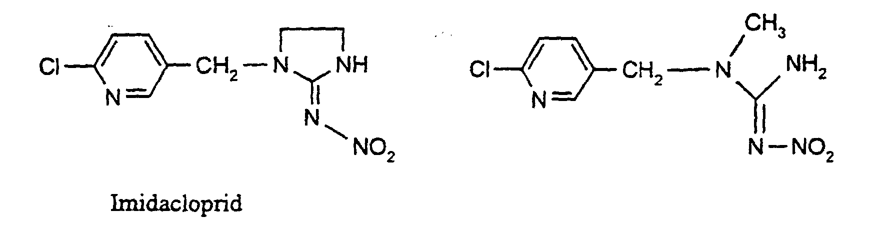

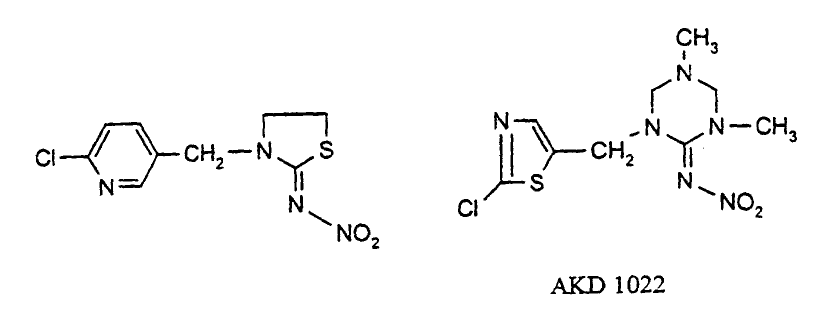

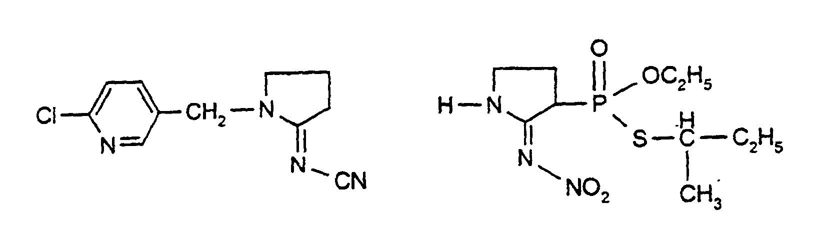

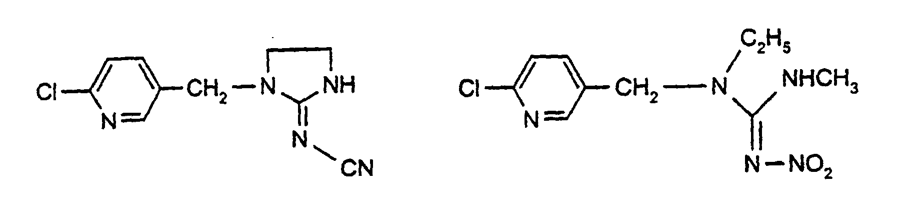

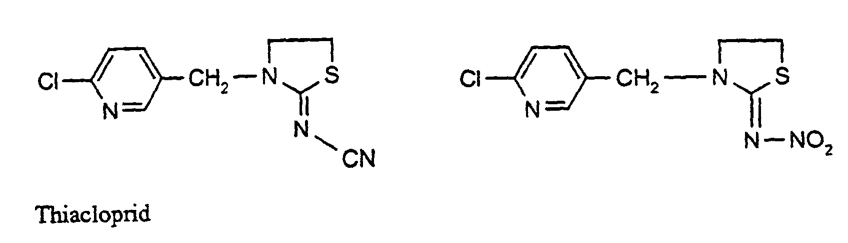

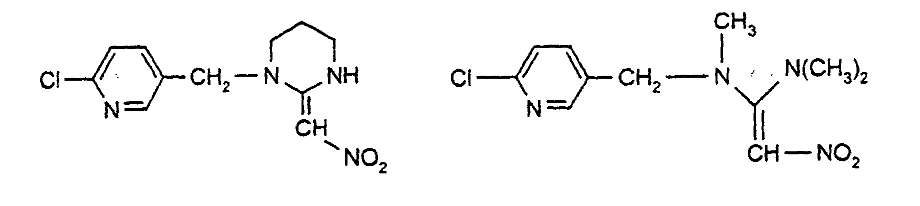

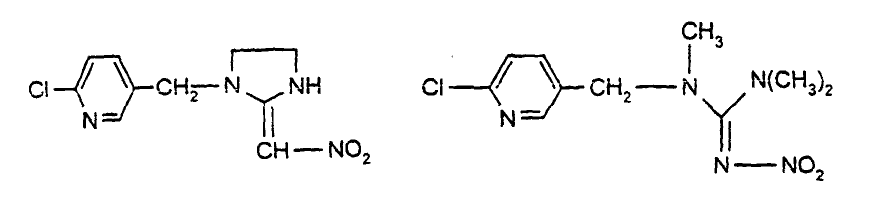

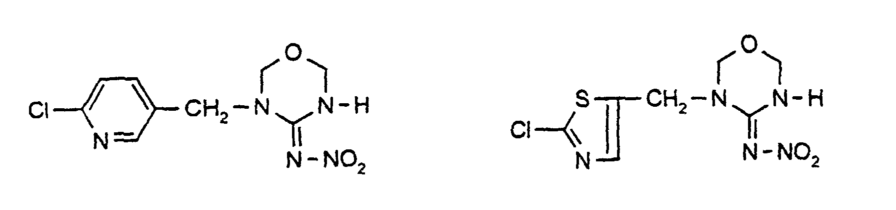

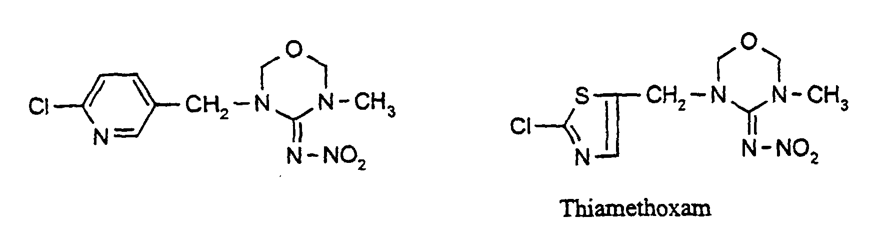

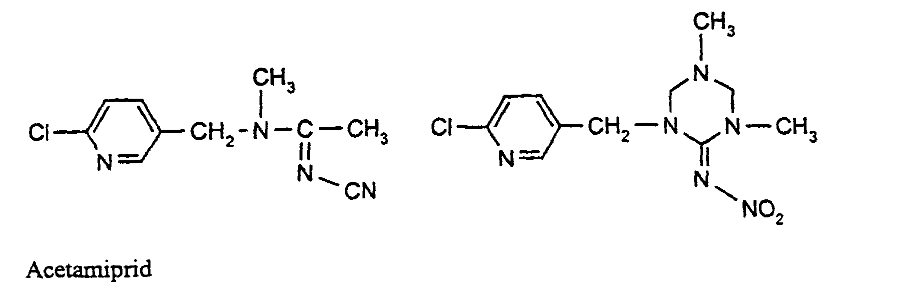

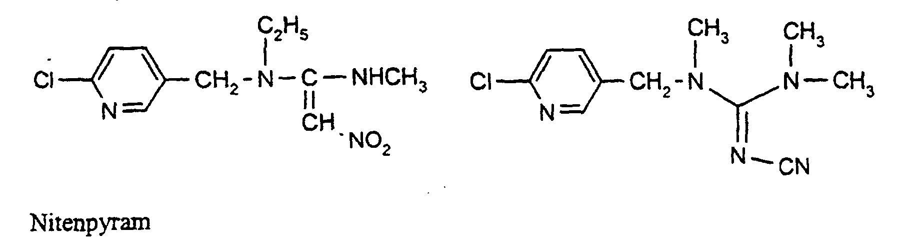

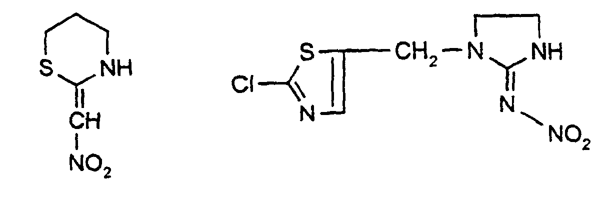

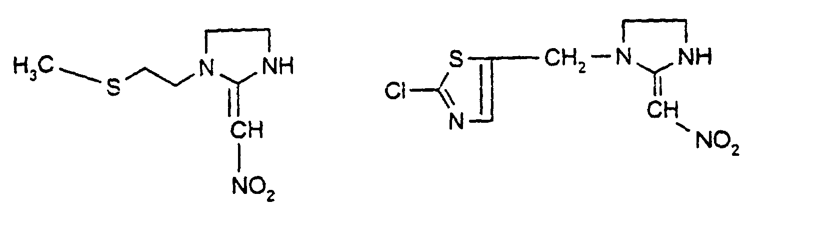

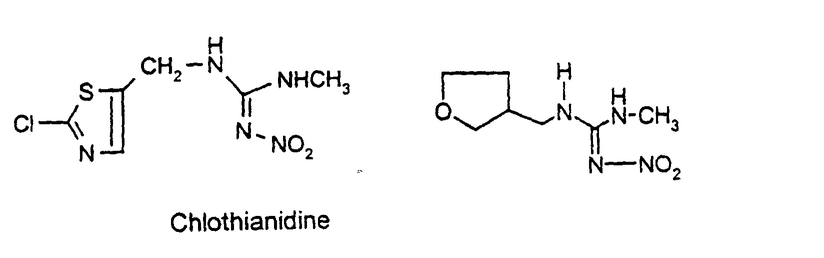

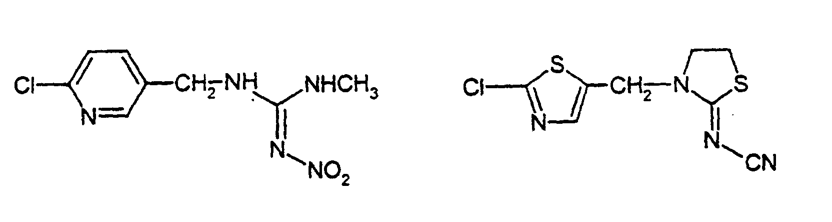

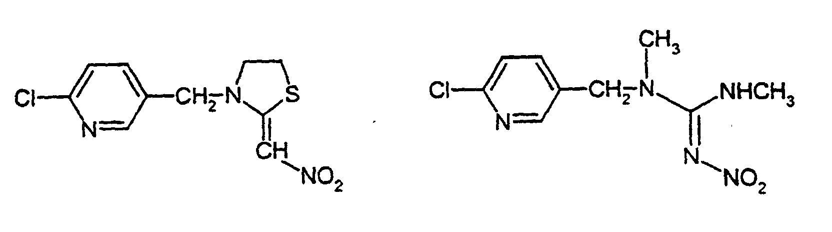

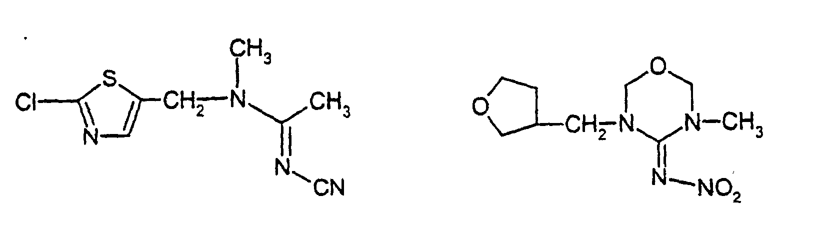

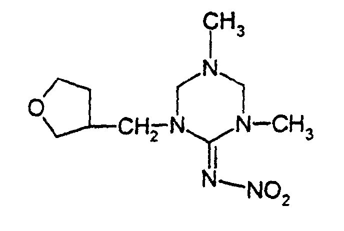

Besonders hervorgehoben seien die Verbindungen

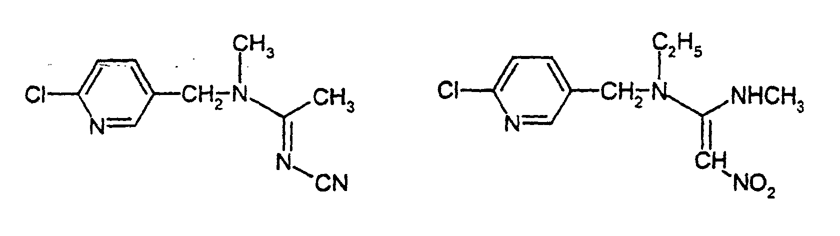

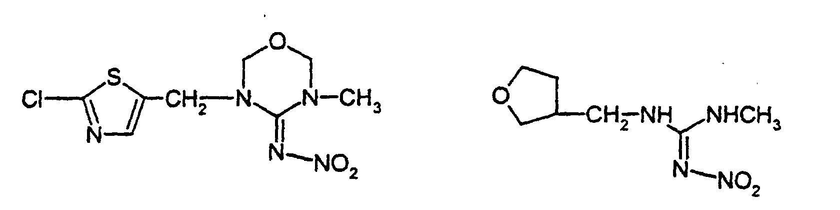

Weiterhin besonders hervorgehoben seien die Verbindungen

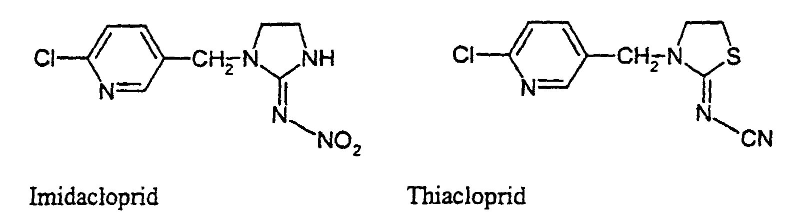

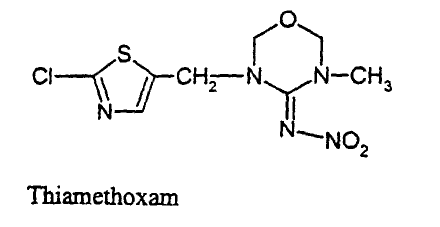

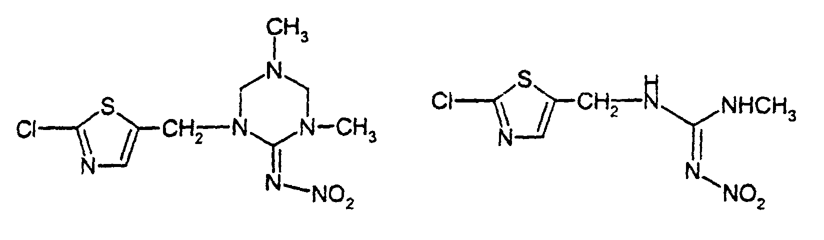

Als Carbamate seien genannt substituierte Phenyl- und Naphthylcarbamate.

Als Phenylpyrazole seien zum Beispiel die folgenden Verbindungen genannt:

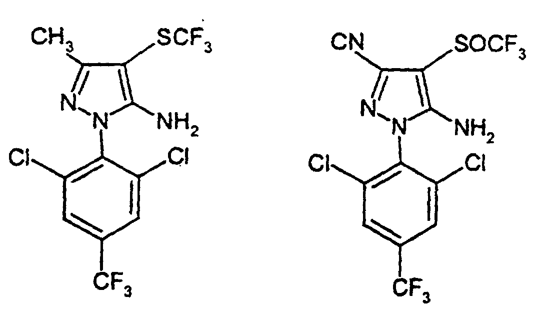

Bevorzugt seien genannt:
- 2-Oxobutylphenyl-N-methylcarbamat,
- 4-Dimethylamino-3-methyl-phenyl-N-methylcarbamat,
- 2-isopropoxy-phenyl-N-methylcarbamat,
- 1-Naphthyl-N-methylcarbamat,
- m-Tolyl-N-methylcarbamat,
- 3,4-Xylyl-N-methylcarbamat,
- 3,5-Xylyl-N-methylcarbamat,
- 2-[1,3-Dioxolan-2-yl]-phenyl-N-methylcarbamat.

Als Phosphorsäureester seien bevorzugt genannt die Verbindungen mit den Common Names Phoxim, Fenitrothion, Dichlorvos, Trichlorfon und Malathion.

Juvenilhormone und juvenilhormonartige Substanzen wie:

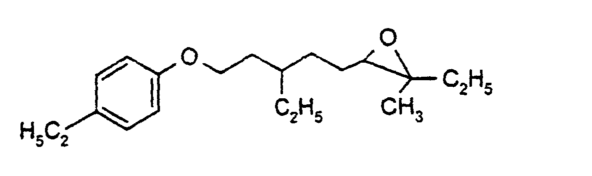

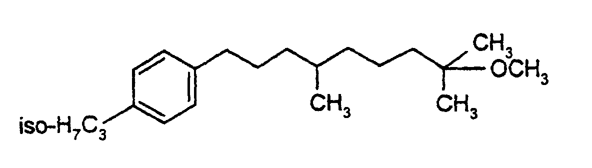

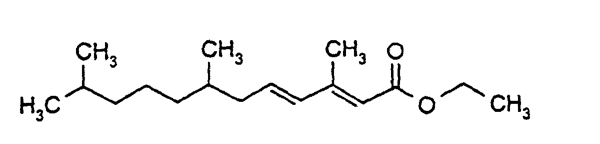

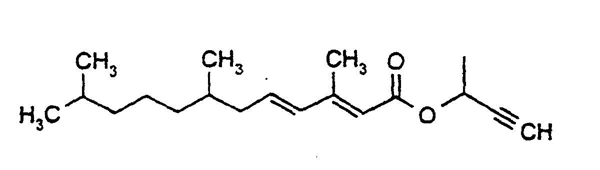

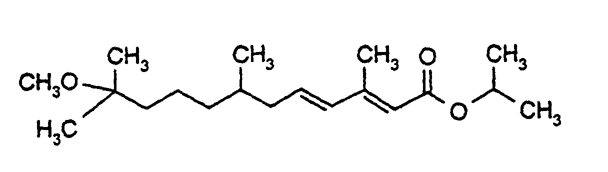

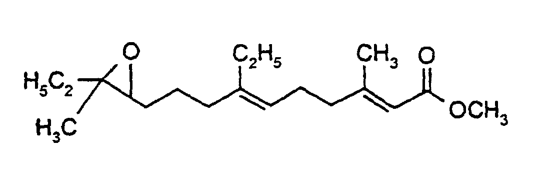

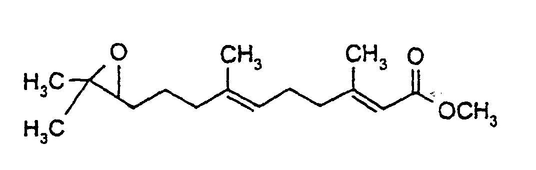

Substituierte Diarylether wie:

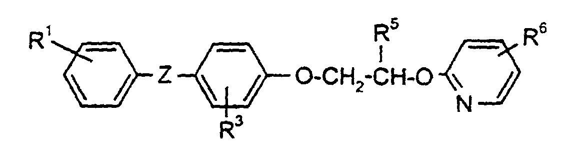

| R¹ | R³ | R⁵ | R⁶ | Z |
|---|---|---|---|---|
| H | H | CH₃ | 2-Cl | O |
| 5-F | H | CH₃ | H | O |
| H | H | CF₃ | H | O |
| H | H | C₂H₅ | H | O |
| H | H | H | H | O |
| H | H | CH₃ | H | CH₂ |
| H | H | CH₃ | H | C(CH₃)₂ |

Benzoylharnstoffe wie:

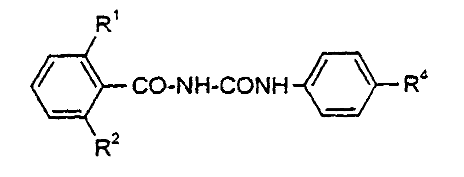

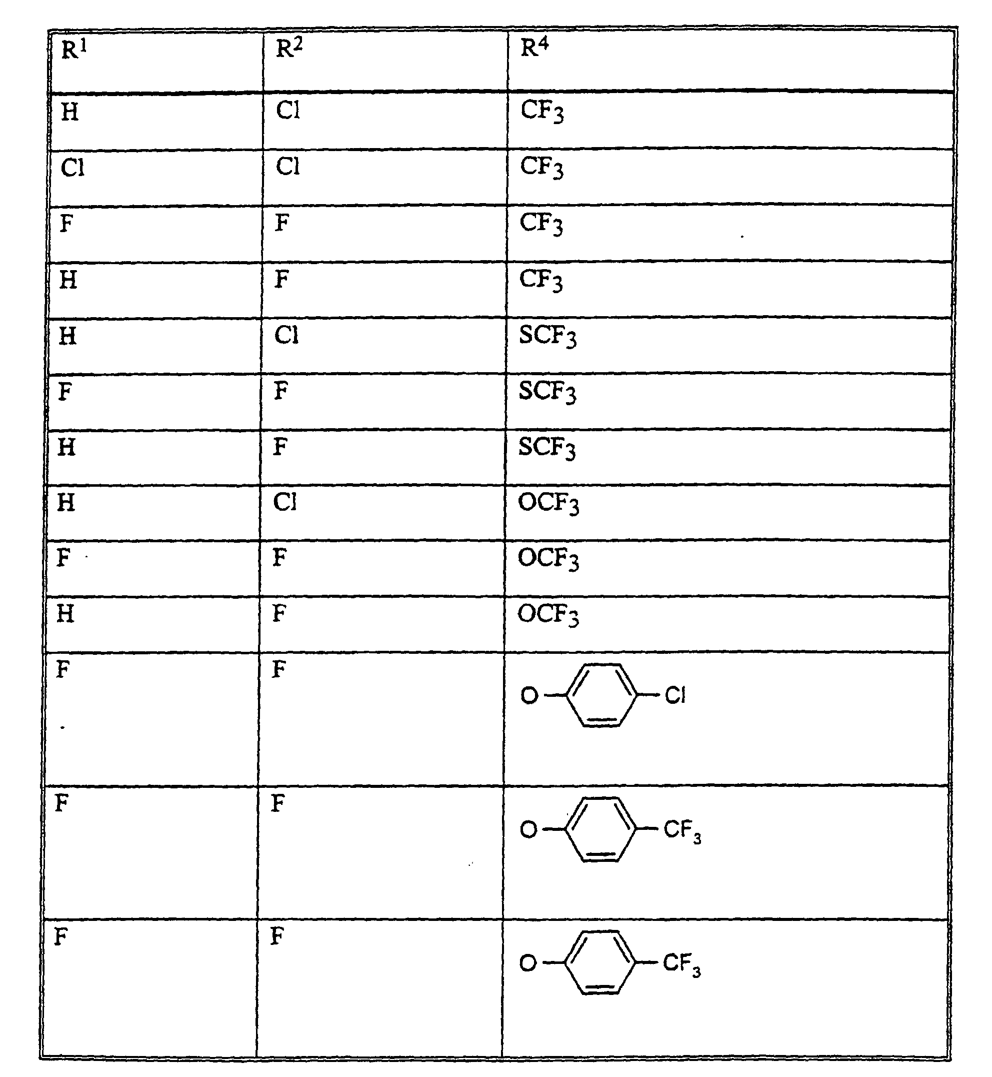

Triazine wie:

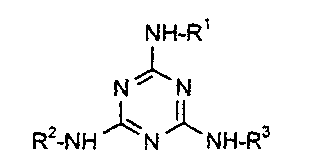

| R¹ | R² | R³ |
|---|---|---|
| Cyclopropyl | H | H |
| Cyclopropyl | H | CH₃ |
| Cyclopropyl | H | C₂H₅ |
| Cyclopropyl | H | C₃H₇-n |
| Cyclopropyl | H | C₄H₉-n |
| Cyclopropyl | H | C₅H₁₁-n |
| Cyclopropyl | H | C₆H₁₃-n |
| Cyclopropyl | H | C₇H₁₅-n |
| Cyclopropyl | H | C₈H₁₇-n |
| Cyclopropyl | H | C₁₂-H₂₅-n |
| Cyclopropyl | H | CH₂-C₄H₉-n |
| Cyclopropyl | H | CH₂CH(CH₃)C₂H₅ |
| Cyclopropyl | H | CH₂CH=CH₂ |
| Cyclopropyl | Cl | C₂H₅ |
| Cyclopropyl | Cl | C₆H₁₃-n |
| Cyclopropyl | Cl | C₈H₁₇-n |
| Cyclopropyl | Cl | C₁₂H₂₅-n |
| Cyclopropyl | H | Cyclopropyl |
| Cyclopropyl | H | COCH₃ |
| Cyclopropyl | H | COCH₃ HCl |
| Cyclopropyl | H | COC₂H₅ HCl |
| Cyclopropyl | H | COC₂H₅ |
| Cyclopropyl | H | COC₃H₇-n |
| Cyclopropyl | H | COC₃H₇-i |
| Cyclopropyl | H | COC₄H₉-t HCl |
| Cyclopropyl | H | COC₄H₉-n |
| Cyclopropyl | H | COC₆H₁₃-n |
| Cyclopropyl | H | COC₁₁-H₂₃-n |
| Cyclopropyl | COCH₃ | COC₂H₅ |
| Cyclopropyl | COC₃H₇-n | COC₆H₁₃-n |
| Cyclopropyl | COCH₃ | COC₃H₇-n |
| Cyclopropyl | COC₂H₅ | COC₃H₇-n |
| Cyclopropyl | H | COCyclopropyl |
| Cyclopropyl | COCyclopropyl | COCyclopropyl |
| Cyclopropyl | COCH₃ | COCH₃ |
| Isopropyl | H | H |
| Isopropyl | H | COCH₃ |
| Isopropyl | H | COC₃H₇-n |
| Cyclopropyl | H | CONHCH₃ |
| Cyclopropyl | H | CONHC₃H₇-i |
| Cyclopropyl | CONHCH₃ | CONHCH₃ |
| Cyclopropyl | H | SCNHCH₃ |
| Cyclopropyl | H | CONHCH₂CH=CH₂ |
| Cyclopropyl | CONHCH₂CH=CH₂ | CONHCH₂CH=CH₂ |
| Cyclopropyl | CSNHCH₃ | CSNHCH₃ |

Die Mengen der Kombinationswirkstoffe können zwischen 0,1 bis 12,5 % breit variiert werden, wobei die Mengen im Bereich 0,1 bis 10,0 % besonders und die Mengen im Bereich 0,5 bis 7,5 % ganz besonders zu bevorzugen sind. Prozentangaben sind hier als Gewichtsprozente zu verstehen.

Besonders bevorzugt sind Kombinationen der Pyrethroide und Pyrethrine, insbesondere Flumethrin, mit Chloronicotinylen, insbesondere Imidacloprid, Thiamethoxam, Chlothianidin, Nitenpyram, Acetamiprid und Thiacloprid.

Als Synergist für diese Verbindungen seien bevorzugt genannt Piperonylbutoxid und Sesamöl. Diese Synergisten sind beispielsweise in der Patentschrift EP-413 610 beschrieben.

Die ausgewählten Formulierungshilfsmittel auf Basis von Polydimethylsiloxanen mit kationischen Quarternären-Amingruppen der Formel 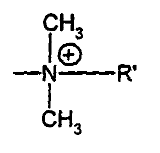 R' = unterschiedliche organische Reste
sind bekannte polymere oder oligomere Verbindungen. Beispielhaft, aber nicht einschränkend seien die in EP-A 0 017 121, S. 2, Z. 11 bis S. 3, Z. 3, EP-A 0 017 122, S. 2, Z. 11 bis S. 3, Z. 13, EP-A 0 166 122, S. 4, Z. 31 bis S. 7 ganz, EP-A 0 294 642, S. 5, Z. 10 bis S. 8, Z. 51, EP-A 282 720, S. 6, Z. 10 bis S. 14, Z. 54 sowie die in EP-A 0 164 668 auf S. 4, Z. 31 bis S. 8, Z. 3 beschriebenen Polysiloxane genannt.

Zur Herstellung der erfindungsgemäßen Mittel können Polysiloxane sowohl mit Mono- als auch mit Polyquartemären- Amingruppen eingesetzt werden. Selbstverständlich können die besagten Polydimethylsiloxane weitere funktionale Gruppen wie Carbonsäure, Amin, Hydroxyl, Carbonsäureester aufweisen. Die ganz besonders bevorzugten funktionellen Gruppen sind Hydroxyl und Carbonsäuren. Ihre Viskosität kann im Bereich 200 bis 17 500 mm²s⁻¹ (bei 25°C), gemessen entsprechend DIN 53 019 als 50 %ige, wässrige Lösung, breit variiert werden, wobei die mit der Viskosität im Bereich 250 bis 10 000 mm²s⁻¹ (bei 25°C) besonders, sowie die mit der Viskosität im Bereich 250 bis 1 350 mm²s⁻¹ (bei 25°C) ganz besonders zu bevorzugen sind.

Ganz besonders bevorzugt sind Polysiloxane der Formel (V) 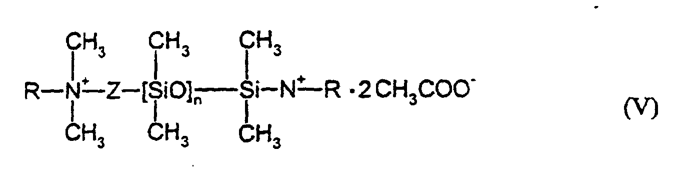
- Z=: 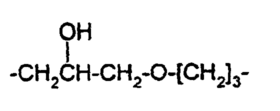
- R =: langkettiger Alkylrest,
die unter dem Handelsnamen ABIL® von der Firma Goldschmidt vertrieben werden.

Die eingesetzten Polydimethylsiloxan-mengen können zwischen 0,1- 15 % breit variiert werden. Wobei die Mengen im Bereich 0,1-7,5 % zu bevorzugen sind. Die Mengen im Bereich 0,25 - 2,5 % werden zur Herstellung der erfindungsgemäß neuen Formulierungen besonders bevorzugt eingesetzt. Die Prozentangaben sind hierbei als Gewichtsprozente zu verstehen.

Als erfindungsgemäße Lösungsmittel werden aliphatische Polyether wie Diethylenglykolmonomethylether, Dipropylenglykolmonopropylether, cyclische Carbonate wie Propylencarbonat, Ethylencarbonat, aliphatische und aromatische Alkohole wie Ethanol, Isopropanol Acetate wie Benzylacetat, Benzylbenzat bzw. deren Mischungen untereinander eingesetzt.

Besonders bevorzugt als Lösungsmittel sind Diethylenglykolmonomethylether, Dipropylenglykolmonopropylether, Propylencarbonat, Ethanol und Isopropanol. Diethylenglykolmonomethylether, Dipropylenglykolmonopropylether, Ethanol und Isopropanol bzw. deren Mischungen untereinander werden zur Herstellung der neuen Flüssigformulierungen ganz besonders bevorzugt eingesetzt.

In der erfindungsgemäßen Formulierung liegt der organische Lösungsmittelanteil bei 2,5 bis 99,8 Gew.-%, bevorzugt bei 75 bis 99,0 Gew.-%, ganz besonders bevorzugt bei 80 bis 92,5 Gew.-%.

Der Anteil an Wasser in den neuen Formulierungen kann durch 0-95 Gew.-% breit variiert werden, wobei 0-15 Gew.-% besonders, 5-17,5 Gew-% ganz besonders zu bevorzugen sind.

Zusätzlich können die erfindungsgemäßen Formulierungen übliche Hilfsmittel wie Antioxidantien oder Geruchsmaskierungsmittel enthalten.

Als Stabilisatoren und Antioxidantien seien genannt Sulfite oder Metabisulfite wie Kaliummetabisulfit; organische Säuren wie Citronensäure, Ascorbinsäure; Phenole, Butylhydroxytoluol, Butylhydroxyanisol, Tocopherol. Wobei die organischen Säuren Citronensäure und Äpfelsäure zu bevorzugen sind. Ganz besonders bevorzugte Stabilisatoren sind Citronensäure und Butylhydroxytoluol. Ihr Anteil kann im Bereich 0,05 bis 2,5 Gew.-% breit variiert werden. Wobei die Mengen im Bereich 0,075 - 0,15 Gew.-% besonders bevorzugt werden.

Geruchsmaskierungsmittel sind z.B. Mischungen organischer Fettsäureester. Sie sind bevorzugt zu 0,1 bis 2 Gew.-% in den erfindungsgemäßen Formulierungen enthalten.

Überraschenderweise zeichnen sich die erfindungsgemäßen Flüssigformulierungen durch eine hervorragende Lagerungsstabilität von mehreren Jahren in allen Klimazonen, Haut-, Anwender- sowie Umweltverträglichkeit aus. Sie eignen sich erstaunlicherweise auch hervorragend zum Abfüllen und Ausbieten in lagerungskritischen "Single dose Polypropylenkunststofftuben" der Wandstärke von 300-500 µm und des Abfüllvolumens 1,0 bis 4,0 ml.

Solche mit den erfindungsgemäßen Mitteln befüllte Single-Dose-Polypropylenkunststofftube sind daher ebenfalls Gegenstand der vorliegenden Erfindung.

Die erfindungsgemäßen Flüssigformulierungen zeigen zudem einen nicht zu erwartenden synergistischen, d.h. wirkungssteigemden Effekt. Beispielsweise bei Verwendung von Pyrethroiden/Pyrethtinen als Wirkstoffes.

Die erfindungsgemäßen Mittel sind umweltverträglich und aufgrund der sehr geringen Toxizität anwenderfreundlich.

Die erfindungsgemäßen Mittel eignen sich bei günstiger Warmblütertoxizität zur Bekämpfung von parasitierenden Insekten, die in der Tierhaltung und Tierzucht bei Haus- und Nutztieren sowie Zoo-, Labor-, Versuchs- und Hobbytieren vorkommen. Sie sind dabei gegen alle oder einzelne Entwicklungsstadien der Schädlinge sowie gegen resistente und normal sensible Arten der Schädlinge wirksam.

Zu den Schädlingen gehören:
Aus der Ordnung der Anoplura z.B. Haematopinus spp., Linognathus spp., Solenopotes spp., Pediculus spp., Pthirus spp.;
aus der Ordnung der Mallophaga z.B. Trimenopon spp., Menopon spp., Eomenacanthus spp., Menacanthus spp., Trichodectes spp., Felicola spp., Damalinea spp., Bovicola spp;
aus der Ordnung der Diptera z.B. Chrysops spp., Tabanus spp., Musca spp., Hydrotaea spp., Muscina spp., Haematobosca spp., Haematobia spp., Stomoxys spp., Fannia spp., Glossina spp., Lucilia spp., Calliphora spp., Auchmeromyia spp., Cordylobia spp., Cochliomyia spp., Chrysomyia spp., Sarcophaga spp., Wohlfartia spp., Gasterophilus spp., Oesteromyia spp., Oedemagena spp., Hypoderma spp., Oestrus spp., Rhinoestrus spp., Melophagus spp., Hippobosca spp..

Aus der Ordnung der Siphonaptera z.B. Ctenocephalides spp., Echidnophaga spp., Ceratophyllus spp..

Besonders hervorgehoben sei die Wirkung gegen Siphonaptera, insbesondere gegen Flöhe und Zecken.

Zu den Nutz- und Zuchttieren gehören Säugetiere wie z.B. Rinder, Pferde, Schafe, Schweine, Ziegen, Kamele, Wasserbüffel, Esel, Kaninchen, Damwild, Rentiere, Pelztiere wie z.B. Nerze, Chinchilla, Waschbär, Vögel wie z.B. Hühner, Gänse, Puten, Enten.

Zu Labor- und Versuchstieren gehören Mäuse, Ratten, Meerschweinchen, Goldhamster, Hunde und Katzen.

Zu den Hobbytieren gehören Hunde und Katzen.

Insbesondere hervorgehoben sei die Anwendung bei Katze und Hund.

Die Anwendung kann sowohl prophylaktisch als auch therapeutisch erfolgen.

Zur Herstellung der erfindungsgemäßen Formulierung werden die gewünschten Bestandteile in entsprechenden Mengen miteinander vermischt, z.B. durch den Einsatz konventioneller Rührkessel oder anderer geeigneter Geräte.

Falls die Inhaltsstoffe es erfordern, kann auch unter Schutzatmosphäre oder anderen Methoden des Sauerstoffausschlusses gearbeitet werden.

Die nachfolgenden Beispiele sollen die Erfindung erläutern:

### Beispiel 1

Eine homogene Pump Spray Formulierung (100 ml) bestehend aus

| | |
|---|---|
| 0,30 g | Flumethrin |
| 27,05 g | Diethylenglykolmonoethylether |
| 50,50 g | Isopropanol |
| 7,50 g | Wasser |
| 1,00 g | Abil Quat 3272 (1) |
| 0,1 g | Citronensäure |
| 0,6 g | Marena PH 99000 |

| | |
|---|---|
| (1) Abil Quat 3272, 50 %ige Ware- ist eine 50 %ige wässrige Polydimethylsiloxan-lösung mit Diquatemärer Ammoniumgruppe und dem Viskositätsbereich von 1000 +/- 200 [mm².s-1] bei 25 ° C der Fa.Goldschmidt AG D-4300 Essen | |

In einem Placebo-kontrollierten Laborversuch (PLRS 9806) zur Wirksamkeit der unter Beispiel 1 genannten Formulierung wurden insgesamt 20 Hunde (verschiedene Rassen, männlich und weiblich) eingesetzt. Alle Tiere wurden zweimalig am Tag -5 und -1 vor Behandlung mit jeweils 60 Zecken (*Rhipicephalus sanguineus*, *Dermacentor variabilis*) infestiert. Am Tag der Behandlung wurden die an den Hunden parasitierenden Zecken gezählt und die Hunde anhand der Parasitenzahl in eine Rangordnung gebracht. Alle Tiere wurden alternierend einer der beiden Versuchsgruppen zugeordnet. Die Tiere der Versuchsgruppe 1 erhielten ca. 3 ml der unter Beispiel 1 genannten Formulierung pro Kilogramm Körpergewicht. Die Tiere der Versuchsgruppe 2 wurden mit ca. 3 ml einer Placeboformulierung pro Kilogramm Körpergewicht behandelt. Die Applikation erfolgte in beiden Gruppen durch Einsprühen der gesamten Körperoberfläche.

Die Prüfung auf Wirksamkeit auf bereits parasitierende Zecken erfolgte durch erneutes Zählen der Zecken in jedem Hund an den beiden darauf folgenden Tagen. Dabei wurde festgestellt, daß die Wirksamkeit der unter Beispiel 1 genannten Formulierung gegenüber der Placeboformulierung am 2. Tag nach Behandlung 96.3% betrug.

Die Prüfung auf Langzeitwirksamkeit (Protektion vor Neuinfestation) erfolgte durch wöchentliche Reinfestation mit jeweils 60 Zecken pro Tier. Dabei konnte nachgewiesen werden, daß sich in beiden Versuchsgruppen über mindestens 5 Wochen keine oder nur einzelne Zecken ansaugen. Dabei waren die Formulierungen gut verträglich.

### Beispiel 2

Eine homogene Pump Spray Formulierung (100 ml) bestehend aus

| | |
|---|---|
| 0,30 g | Flumethrin |
| 27,65 g | Diethylenglykolmonoethylether |
| 50,50 g | Isopropanol |
| 7,50 g | Wasser |
| 1,00 g | Abil Quat 3274 (2) |
| 0,1 g | Citronensäure |

| | |
|---|---|
| (2) Abil Quat 3274 ist eine 50 % ige wässrige Polydimethylsiloxan-Lösung mit Diquaternäre Amoniumgruppe und dem Viskositätsbereich von 5000 - 15000 [mm².s-1] bei 25°C der Fa.Goldschmidt AG D-4300 Essen | |

In einem kontrollierten Laborversuch (GZ 49/97) zur Wirksamkeit der unter Beispiel 2 genannten Formulierung wurden insgesamt 10 Hunde (Retriever, Labradors, Setter, männlich und weiblich) eingesetzt. Alle Tiere wurden einmalig am Tag -3 vor Behandlung mit jeweils 70 Zecken (*Rhipicephalus sanguineus, Ixodes canisuga*) infestiert. Am Tag der Behandlung wurden die an den Hunden parasitierenden Zecken gezählt und die Hunde anhand der Parasitenzahl in eine Rangordnung gebracht. Alle Tiere wurden alternierend einer der beiden Versuchsgruppen zugeordnet. Die Tiere der Versuchsgruppe 1 erhielten ca. 3 ml der unter Beispiel 2 genannten Formulierung pro Kilogramm Körpergewicht. Die Tiere der Versuchsgruppe 2 blieben unbehandelt. Die Applikation erfolgte in Versuchsgruppe 1 durch Einsprühen der gesamten Körperoberfläche.

Die Prüfung auf Wirksamkeit auf bereits parasitierende Zecken erfolgte durch erneutes Zählen der Zecken in jedem Hund an den drei darauf folgenden Tagen. Dabei wurde festgestellt, daß die Wirksamkeit der unter Beispiel 2 genannten Formlierung gegenüber der unbehandelten Kontrollgruppe am 3. Tag nach Behandlung 100% gegen beide Zeckenarten betrug.

Die Prüfung auf Langzeitwirksamkeit (Protektion vor Neuinfestation) erfolgte durch wöchentliche Reinfestation mit jeweils 70 Zecken pro Tier. Dabei konnte nachgewiesen werden, daß sich in beiden Versuchsgruppen über 5 bis 7 Wochen keine oder nur einzelne Zecken ansaugten. Dabei war die Formulierung gut verträglich.

### Beispiel 3

Eine homogene Pump Spray Formulierung (100 ml) bestehend aus

| | |
|---|---|
| 0,30 g | Flumethrin |
| 27,05 g | Dipropylenglykolmonomethylether |
| 50,50 g | Ethanol |
| 7,50 g | Wasser |
| 1,00 g | Abil Quat 3272 (1) |
| 0,1 g | Citronensäure |
| 0,6 g | Marena PH 99000 |

Aus den Laborprüfungen zur Zecken-Wirksamkeit gemäß Beispiel 2 geht hervor, daß die Formulierung eine sehr gute Wirkung gegen Zecken aufweist.

### Beispiel 4

Eine homogene Pump Spray Formulierung (100 ml) bestehend aus

| | |
|---|---|
| 0,30 g | Flumethrin |
| 27,35 g | Diethylenglykolmonoethylether |
| 50,50 g | Isopropanol |
| 7,50 g | Wasser |
| 1,00 g | Abil Quat 3272 (1) |
| 0,1 g | Citronensäure |
| 0,3 g | Imidacloprid |

Aus den Laborprüfungen zur Zecken-Wirksamkeit gemäß Beispiel 2 geht hervor, daß die Formulierung eine sehr gute Wirkung gegen Zecken aufweist und zum Bekämpfen von Zecken gut geeignet ist.

Zur Ermittlung der Floh-Wirksamkeit der Formulierung wurden insgesamt 10 Hunde (Retriever, Labradors, Setter, männlich und weiblich) eingesetzt. Alle Tiere wurden einmalig am Tag -3 vor Behandlung mit jeweils 100 Flöhen infestiert. Am Tag der Behandlung wurden die an den Hunden parasitierenden Flöhe gezählt und die Hunde anhand der Parasitenzahl in eine Rangordnung gebracht. Alle Tiere wurden alternierend einer der beiden Versuchsgruppen zugeordnet. Die Tiere der Versuchsgruppe 1 erhielten ca. 3 ml der unter Beispiel 2 genannten Formulierung pro Kilogramm Körpergewicht. Die Tiere der Versuchsgruppe 2 blieben unbehandelt. Die Applikation erfolgte in Versuchsgruppe 1 durch Einsprühen der gesamten Körperoberfläche.

Die Prüfung auf Wirksamkeit auf bereits parasitierende Flöhe erfolgte durch erneutes Zählen der Flöhe in jedem Hund an den darauf folgenden Tagen. Dabei wurde festgestellt, daß die Wirksamkeit der unter Beispiel 4 genannten Formlierung gegenüber der unbehandelten Kontrollgruppe am zweiten Tag nach Behandlung 100% gegen Flöhe betrug. Nun wurden die Tiere am Tage 7,14, 21 und 28 nach der Behandlung mit je 100 Flöhen infestiert. Jeweils am Tage 8, 15, 22 und 29 nach der Behandlung wurden die am Hund verbliebenen Flöhe gezählt. Es konnten keine lebenden Flöhe gefunden werden. Die Floh-Wirkung war 100 %.

### Beispiel 5

Eine homogene Pump Spray Formulierung (100 ml) bestehend aus

| | |
|---|---|
| 0,30 g | Flumethrin |
| 27,35 g | Diethylenglykolmonoethylether |
| 50,50 g | Isopropanol |
| 7,50 g | Wasser |
| 1,00 g | Abil Quat 3272 (1) |
| 0,1 g | Citronensäure |
| 0,3 g | Pyriproxyfen |

Aus den Laborprüfungen zur Zecken-Wirksamkeit gemäß Beispiel 2 geht hervor, daß die Formulierung eine gute Wirkung gegen Zecken aufweist und zum Bekämpfen von Zecken gut geeignet ist. Ferner konnte nachgewiesen werden, daß sich die Formulierung zum Bekämpfen von Flohlarven sehr gut eignet. Die Flohlarvenwirkung war bis zu 45 Tage nach der Behandlung 100 %.

### Beispiel 6

Eine homogene Spot on Formulierung (100 ml) bestehend aus

| | |
|---|---|
| 2,00 g | Flumethrin |
| 81,00g | Diethylenglykolmonoethylether |
| 15,00 g | Wasser |
| 2,00 g | Abil Quat 3272 (1) |
| 0,1 g | Citronensäure |
| 0,1 g | BHT (Butylhydroxytoluol) |

### Beispiel 7

Eine homogene Spot on Formulierung (100 ml) bestehend aus

| | |
|---|---|
| 2,00 g | Flumethrin |
| 76,00g | Diethylenglykolmonoethylether |
| 15,00 g | Wasser |
| 2,00 g | Abil Quat 3272 (1) |
| 5,00 g | Piperonylbutoxid |
| 0,1 g | Citronensäure |
| 0,1 g | BHT (Butylhydroxytoluol) |

In einem nicht kontrollierten Laborversuch (GZ 35/99) zur Wirksamkeit der unter Beispiel 6 und 7 genannten Formulierungen wurden insgesamt 15 Hunde (Beagle, männlich und weiblich) eingesetzt. Alle Tiere wurden einmalig am Tag -2 vor Behandlung mit jeweils 50 Zecken (*Rhipicephalus sanguineus*) infestiert. Am Tag der Behandlung wurden die an den Hunden parasitierenden Zecken gezählt und die Hunde anhand der Parasitenzahl in eine Rangordnung gebracht. Alle Tiere wurden alternierend einer der beiden Versuchsgruppen zugeordnet. Die Tiere der Versuchsgruppe 1 erhielten 0.4 ml der unter Beispiel 7 genannten Formulierung pro Kilogramm Körpergewicht. Die Tiere der Versuchsgruppe 2 wurden mit 0.4 ml der unter Beispiel 6 genannten Formulierung pro Kilogramm Körpergewicht behandelt. Die Applikation erfolgte in beiden Gruppen direkt auf die Haut im Nackenbereich und Rückenbereich, wobei die Gesamtdosis zu jeweils 1 ml / Hautstelle (Spot) verabreicht wurde.

Die Prüfung auf Wirksamkeit auf bereits parasitierende Zecken erfolgte durch erneutes Zählen der Zecken in jedem Hund an den beiden darauf folgenden Tagen. Dabei wurde festgestellt, daß durch die unter Beispiel 7 genannten Formlierung am 2. Tag nach Behandlung 64% aller angesaugten Zecken getötet wurden. Bei Verwendung der unter Beispiel 6 genannten Formulierung waren es entsprechend 73%.

Die Prüfung auf Langzeitwirksamkeit (Protektion vor Neuinfestation) erfolgte durch wöchentliche Reinfestation mit jeweils 50 Zecken pro Tier. Dabei konnte nachgewiesen werden, daß sich in beiden Versuchsgruppen über mindestens 6 Wochen keine oder nur einzelne Zecken ansaugen. Dabei waren Placebo- und wirkstoffhaltige Formulierung gut verträglich.

### Beispiel 8

Eine wäßrige, homogene Emulsion-Spot on Formulierung (100 ml) bestehend aus

| | |
|---|---|
| 2,00 g | Flumethrin |
| 4,00g | Diethylenglykolmonoethylether |
| 63,45 g | Wasser |
| 10,00 g | Abil Quat 3272 (1) |
| 30,00 g | Harnstoff |
| 0,1 g | Citronensäure |

Aus den Laborprüfungen zur Zecken-Wirksamkeit gemäß den Beispielen 6 und 7 geht hervor, daß die Formulierung eine gute Wirkung gegen Zecken aufweist und zum Bekämpfen von Zecken gut geeignet ist.

### Beispiel 9

Eine homogene Spot on Formulierung (100 ml) bestehend aus

| | |
|---|---|
| 1,00 g | Flumethrin |
| 5,00 g | Piperonylbutoxid |
| 2,50 g | Imidacloprid |
| 15,00 g | Wasser |
| 2,00 g | Abil Quat 3272 (1) |
| 74,77 g | Diethylenglykolmonoethylether |
| 0,13 g | Citronensäure |
| 0,10 g | BHT (Butylhydroxytoluol) |

wurde zur Durchführung von Wirksamkeitsuntersuchungen bei Hunden gegen Zecken sowie gegen Flöhe gemäß Beispiel 4 eingesetzt. Das Applikationsvolumen lag bei 0,4 ml/kg Körpergewicht. Die Floh- und Zeckenwirkung der Formulierung lag bei 100 % mit einer Langzeitwirkung von mindestens 5 Wochen.

Aus den analytischen Streßuntersuchungen geht hervor, dass die erfindungsgemäße Formulierung zum Abfüllen in Single-Dose-PP-Tuben mit einem Gesamtabfüllvolumen von < 10 ml und einer Wandstärke von - 350 µm hervorragend geeignet ist und in allen Klimazonen eine Langzeitstabilität von mind. 36 Monaten aufweist.

### Beispiel 10

Eine homogene Spot on Formulierung (100 ml) bestehend aus

| | |
|---|---|
| 1,00 g | Flumethrin |
| 5,00 g | Piperonylbutoxid |
| 2,50 g | Chlothianidine |
| 15,00 g | Wasser |
| 2,00 g | Abil Quat 3272 (1) |
| 74,77 g | Diethylenglykolmonoethylether |
| 0,13 g | Citronensäure |
| 0,10 g | BHT (Buthylhydroxytoluol) |

wurde zur Durchführung von Wirksamkeitsuntersuchungen bei Hunden gegen Zecken sowie gegen Flöhe gemäß Beispiel 4 eingesetzt. Die Applikationsvolumen lag bei 0,4 ml/kg Körpergewicht. Die Floh- und Zeckenwirkung der Formulierung lag bei 100 % mit einer Langzeitwirkung von mind. 5 Wochen. Die Hautverträglichkeit der neuen Formulierung ist hervorragend.

Aus den analytischen Streßuntersuchungen geht hervor, dass die erfindungsgemäße Formulierung zum Abfüllen in Single-Dose-PP-Tuben mit einem Gesamtabfüllvolumen von < 10 ml und einer Wandstärke von ~ 350 µm hervorragend geeignet ist und in allen Klimazonen eine Langzeitstabilität von mindestens 36 Monaten aufweist.

### Beispiel 11

Eine homogene Spot on Formulierung (100 ml) bestehend aus

| | |
|---|---|
| 1,00 g | Flumethrin |
| 5,00 g | Piperonylbutoxid |
| 2,50 g | Thiamethoxam |
| 15,00 g | Wasser |
| 2,00 g | Abil Quat 3272 (1) |
| 74,77 g | Diethylenglykolmonoethylether |
| 0,13 g | Citronensäure |
| 0,10 g | BHT (Buthylhydroxytoluol) |
| 0,10 g | Tocopherol (Vit. E) |

wurde zur Durchführung von Wirksamkeitsuntersuchungen bei Hunden gegen Zecken sowie gegen Flöhe gemäß Beispiel 4 eingesetzt. Das Applikationsvolumen lag bei 0,4 ml/kg Körpergewicht. Die Floh- und Zeckenwirkung der Formulierung lag bei 100 % mit einer Langzeitwirkung von mind. 4 Wochen.

Aus den analytischen Streßuntersuchungen geht hervor, dass die erfindungsgemäße Formulierung zum Abfüllen in Single-Dose-PP-Tuben mit einem Gesamtabfüllvolumen von <10ml und einer Wandstärke von ~ 350 µm hervoragend geeignet ist und in allen Klimazonen eine Langzeitstabilität von mindestens 36 Monaten aufweist. Die erfindungsgemäß neue Formulierung ist hautverträglich und irritationsfrei.

### Beispiel 12

Eine homogene Spot on Formulierung (100 ml) bestehend aus

| | |
|---|---|
| 1,00 g | Flumethrin |
| 5,00 g | Piperonylbutoxid |
| 3,50 g | Thiacloprid |
| 15,00 g | Wasser |
| 2,00 g | Abil Quat 3272 (1) |
| 73,77 g | Diethylenglykolmonoethylether |
| 0,13 g | Citronensäure |
| 0,10 g | BHT (Butylhydroxytoluol) |

wurde zur Durchführung von Wirksamkeitsuntersuchungen bei Hunden gegen Zecken sowie gegen Flöhe gemäß Beispiel 4 eingesetzt. Das Applikationsvolumen lag bei 0,30 ml/kg Körpergewicht. Die Floh- und Zeckenwirkung der Formulierung lag bei 100 % mit einer Langzeitwirkung von mindestens 4 Wochen.

Aus den analytischen Streßuntersuchungen geht hervor, dass die erfindungsgemäße Formulierung zum Abfüllen in Single-Dose-PP-Tuben mit einem Gesamtfüllvolumen von < 10 ml und einer Wandstärke von ~ 350 µm hervorragend geeignet ist und in allen Klimazonen eine Langzeitstabilität von mindestens 36 Monaten aufweist. Aus den Verträglichkeitsversuchungen geht hervor, dass die neue Formulierung nicht zu Hautirritation führt.

### Beispiel 13

Eine homogene Spot on Formulierung (100 ml) bestehend aus

| | |
|---|---|
| 2,00 g | Flumethrin |
| 5,00 g | Piperonylbutoxid |
| 4,50 g | Acetamiprid |
| 15,00 g | Wasser |
| 2,00 g | Abil Quat 3272 (1) |
| 71,77 g | Diethylenglykolmonoethylether |
| 0,13 g | Citronensäure |
| 0,10 g | BHT (Buthydroxytoluol) |

wurde zur Durchführung von Wirksamkeitsuntersuchungen bei Hunden gegen Zecken sowie gegen Flöhe gemäß Beispiel 4 eingesetzt. Das Applikationsvolumen lag bei 0,35 ml/kg Körpergewicht. Die Floh- und Zeckenwirkung der Formulierung lag bei 100 % mit einer Langzeitwirkung von mindestens 4 Wochen.

Aus der analytischen Streßuntersuchungen geht hervor, dass die erfindungsgemäße Formulierung zum Abfällen in Single-Dosen-PP-Tuben mit einem Gesamtvolumen von < 10 ml und einer Wandstärke von - 350 µm hervorragend geeignet ist und in allen Klimazonen eine Langzeitstabilität von mindestens 36 Monaten aufweist. Die neue Formulierung zeichnet sich durch ihre sehr gute Hautverträglichkeit aus.

### Beispiel 14

Eine homogene Spot on Formulierung (100 ml) bestehend aus

| | |
|---|---|
| 2,00 g | Flumethrin |
| 5,00 g | Piperonylbutoxid |
| 15,00 g | Nitenpyram |
| 2,00 g | Abil quat 3272 (1) |
| 71,77 g | Diethylenglykolmonoethyleter |
| 0,13 g | Citronensäure |
| 0,10 g | BHT (Butylhydroxytoluol) |

wurde zur durch Durchführung von Wirksamkeitsuntersuchungen bei Hunden gegen Zecken sowie gegen Flöhe gemäß Beispiel 4 eingesetzt. Das Applikationsvolumen lag bei 0,30 ml/kg Körpergewicht. Die Floh- und Zeckenwirkung der Formulierung lag bei 100 % mit einer Langzeitwirkung von mindestens 4 Wochen.

Aus den analytischen Streßuntersuchungen geht hervor, dass die erfindungsgemäße Formulierung zum Abfüllen in Single-Dose-PP-Tuben mit einem Gesamtabfullvolumen von < 10 ml und einer Wandstärke von ~ 350 µm hervorragend geeignet ist und in allen Klimazonen eine Langzeitstabilität von mindestens 36 Monaten aufweist.

## Patentansprüche

1. Verwendung von Polydimethylsiloxanen, enthaltend mindestens eine quartäre Ammoniumgruppe, als Formulierhilfsmittel in Formulierungen akarizider α-Cyanopyrethroide.

2. Mittel, enthaltend
a) ein akarizides α-Cyanopyrethroid und
b) ein Polydimethylsiloxan enthaltend mindestens eine quartäre Ammoniumgruppe pro Molekül,
sowie gegebenenfalls weitere Hilfs- und Trägerstoffe.

3. Mittel gemäß Anspruch 2, enthaltend ein akarizides α-Cyanopyrethroid ausgewählt aus Fenvalerat, Flumethrin, Cyfluthrin, Cypermethrin, Deltamethrin und Fluvalinat.

4. Single-Dose-Polypropylen-Tuben der Wandstärke ~ 350 µm und einem Gesamtabfüllvolumen von < 10 ml, **dadurch gekennzeichnet, dass** sie mit einem Mittel gemäß einem der Ansprüche 2 oder 3 befüllt sind.

5. Mittel gemäß einem der Ansprüche 2 oder 3, **dadurch gekennzeichnet, dass** es als weiteren Wirkstoff ein Chlornicotinyl-Insektizid enthält.

## Claims

1. Use of polydimethylsiloxanes containing at least one quaternary ammonium group as formulation auxiliaries in formulations of acaricidal α-cyanopyrethroids.

2. Composition, comprising
a) an acaricidal α-cyanopyrethroid and
b) a polydimethylsiloxane containing at least one quaternary ammonium group per molecule,
and, if appropriate, further auxiliaries and carriers.

3. Composition according to Claim 2, comprising an acaricidal α-cyanopyrethroid chosen from fenvalerate, flumethrin, cyfluthrin, cypermethrin, deltamethrin and fluvalinate.

4. Single dose polypropylene tubes having a wall thickness of ~350 µm and a total filling volume of < 10 µm, **characterized in that** they are filled with a composition according to either of Claims 2 and 3.

5. Composition according to either of Claims 2 and 3, **characterized in that** it comprises, as further active compound, a chloronicotinyl insecticide.

## Revendications

1. Utilisation de polydiméthylsiloxanes contenant au moins un groupe ammonium quaternaire comme additif de formulation dans des formulations d'α-cyanopyréthroïde acaricide.

2. Agent contenant :
a) un α-cyanopyréthroïde acaricide et
b) un polydiméthylsiloxane contenant au moins un groupe ammonium quaternaire par molécule
ainsi, le cas échéant, que d'autres substances de support ou additifs.

3. Agent selon la revendication 2, contenant un α-cyanopyréthroïde acaricide choisi parmi le fenvalérate, la fluméthrine, la cyfluthrine, la cyperméthrine, la deltaméthrine et le fluvalinate.

4. Tubes en polypropylène à dose unique, d'une épaisseur de paroi de 350 µm et d'un volume de remplissage total < 10 ml, **caractérisés en ce qu'**ils sont remplis avec un agent selon l'une quelconque des revendications 2 ou 3.

5. Agent selon l'une quelconque des revendications 2 ou 3, **caractérisé en ce qu'**il contient, comme autre principe actif, un insecticide chloronicotinyle.
